# FASCICULE DE BREVET EUROPEEN

(11) **EP 2 672 842 B1**
(45) Date de publication et mention de la délivrance du brevet: **03.05.2017**
(21) Numéro de dépôt: 12703303.3
(22) Date de dépôt: 07.02.2012
(51) Int. Cl.: A61K 31/7016, A61P 1/12, A23L 29/30, A23L 33/00, A23L 33/10, A23L 33/135

(54) **GALACTOFRUCTOSE POUR SON EFFET REGULATEUR DU TRANSIT INTESTINAL**
GALAKTOFRUKTOSE FÜR REGULIERENDEN EFFEKT AUF DIE DARMPASSAGE
GALACTOFRUCTOSE FOR THE REGULATORY EFFECT THEREOF ON INTESTINAL TRANSIT

(30) Priorité: 08.02.2011 FR 1151001
(43) Date de publication de la demande: 18.12.2013
(73) Titulaire: Groupe Solactis, 75018 Paris (FR)
(72) Inventeur: RONFARD, Pascal, F-75018 Paris (FR); BAXTER, Guillaume, B-1050 Bruxelles (BE)
(74) Mandataire: Loyer & Abello
(86) Numéro de dépôt international: PCT/EP2012/052068
(87) Numéro de publication internationale: WO 2012/107455

(56) Documents cités:
- EP-A1- 1 825 857
- EP-A1- 2 251 017
- WO-A1-2006/118370
- SEKI N: "Lactulose as a prebiotics, and Enhancement of Calcium and Magnesium Absorption", 20070514 , 14 mai 2007 (2007-05-14), pages 1-41, XP002614002, Extrait de l'Internet: URL:http://lactose.ru/present/1Nobuo_Seki. pdf [extrait le 2010-12-10]
- NAGENDRA R ET AL: "EFFECT OF INCORPORATING LACTULOSE IN INFANT FORMULA ON ABSORPTION AND RETENTION OF NITROGEN, CALCIUM, PHOSPHORUS AND IRON IN RATS", INTERNATIONAL DAIRY JOURNAL, ELSEVIER APPLIED SCIENCE, BARKING, GB, vol. 4, no. 8, 1 janvier 1994 (1994-01-01) , pages 779-788, XP000933640, ISSN: 0958-6946, DOI: 10.1016/0958-6946(94)90007-8
- BALLONGUE J ET AL: "EFFECTS OF LACTULOSE AND LACTITOL ON COLONIC MICROFLORA AND ENZYMATIC ACTIVITY", SCANDINAVIAN JOURNAL OF GASTROENTEROLOGY, INFORMA HEALTHCARE, GB, vol. 32, no. SUPPL. 222, 1 janvier 1997 (1997-01-01), pages 41-44, XP009033566, ISSN: 0036-5521

## Description

L'invention concerne l'effet d'oligosaccharides sur le transit intestinal de mammifères.

On entend par transit intestinal l'opération complexe qu'effectuent les intestins pour transporter le bol alimentaire de l'estomac vers le rectum.

Actuellement, de plus en plus d'êtres humains souffrent de problèmes de santé liés à un dérèglement du transit intestinal. Le dérèglement du transit intestinal peut aussi bien résulter en des temps de transit trop importants (hypotransit) que des temps trop courts (hyper transit).

Les problèmes de santé résultants d'un dérèglement du transit intestinal peuvent varier de simples ballonnements intestinaux à des douleurs aigues, (hypotransit). Dans le cas de transit intestinal trop rapide, une déshydratation peut même survenir. Dans certaines régions du globe il s'agit d'un problème majeur de santé public, qui concerne surtout les enfants et est une des premières causes de mortalité infantile.

Dans les régions les plus développées du globe, les dérèglements du transit intestinal sont aggravés par un mode de vie sédentaire, une alimentation trop pauvre en fibres et par le stress.

L'effet sur le transit intestinal de certains oligosaccharides est connu. Le galactofructose est en effet couramment utilisé pour accélérer le transit intestinal. Toutefois il peut en résulter la transformation d'hypotransit intestinal en hypertransit intestinal (effet «laxatif»). Par exemples, le document EP1825857 décrit un agent pour accélérer le transit intestinal, Le document WO2006118370 décrit une composition pour traiter et prévenir la constipation. Le document EP2251017 décrit une méthode de diagnostic de la constipation. Les documents SEKI.N « Lactulose as a prebiotics, and Enhancement of calcium and magnesium absorption" du 14 mai 2007, NAGENDRA R et al « Effect of incorporating lactulose in infant formula on absorption and retention of nitrogen, calcium, phosphorus and iron in rats » publié dans le Intenational Dairy Journal, Elsevier Applied Science du 8 janvier 1994 et BALLONGUE J et al "Effects of lactulose and lactitol on colonic microflora and enzymatic activity » publié dans le Scandinavian journal of gastroenterology informa healthcare du 1er janvier 1997 décrivent les effets du lactulose sur la flore intestinale.

Dans FR2891439 est décrit un complément alimentaire comprenant un agent d'augmentation du poids des selles et un agent laxatif. L'agent laxatif est de préférence notamment du lactulose. Toutefois, le complément alimentaire dans FR2891439 est destiné à des patients atteints de constipation, voire même de stades avancés de constipation.

L'invention vise à améliorer le bien-être de mammifères généralement sains, par action sur leur flore intestinale.

En conséquence, l'invention concerne une méthode non-thérapeutique pour ralentir le transit intestinal de mammifères sains, par administration d'une composition alimentaire comprenant du galactofructose, ladite composition alimentaire étant absorbée par le mammifère en quantité correspondant à des doses journalières non-moyennées de galactofructose comprises entre 0,1 et 5 g, avantageusement entre 0,1 et 2 g, pendant au moins 30 jours consécutifs, caractérisée en ce que le mammifère, tout en ne présentant aucun signe de maladie, présente néanmoins un transit intestinal légèrement trop rapide, le mammifère ayant un temps de transit intestinal avant absorption, mesuré par scintigraphie, égal à une valeur comprise dans une fourchette de 10 à 20 heures.

Comme l'homme du métier le comprendra sans peine, le terme « moyenner » doit être compris ici dans son acception mathématique, et signifie donc « calculer la moyenne » (réf.: http://en.wiktionary.org/wiki/moyenner). De manière tout aussi évidente, la moyenne à calculer pour définir la posologie conforme à l'invention est la moyenne arithmétique des doses journalières absorbées pendant la période des 30 jours.

Dans l'invention, le mammifère est sain, ne présentant aucun signe de maladie.

Le galactofructose, appelé également lactulose, est un disaccharide formé de l'association de deux molécules monosaccharides, le fructose et le galactose. Sa formule chimique est (4-O-β-D-Galactopyranosyl-(β-D-fructofuranose). Il s'agit d'un produit d'isomérisation du lactose qui tous deux ont la même formule empirique (C₁₂H₂₂O₁₁) et poids moléculaire (342.3).

Le galactofructose, lorsqu'il est absorbé en quantités faibles selon l'invention dans des compositions alimentaires permet une amélioration du bien-être des mammifères. L'invention présente en outre l'avantage de ne pas perturber le goût et l'apparence (par exemple couleur) des compositions alimentaires. Cela est particulièrement intéressant pour les alimentations infantile et animale, dans lesquelles une modification de goût peut provoquer un rejet de l'aliment.

On recommande que le galactofructose dans la méthode selon l'invention comprenne moins de 30%, de préférence moins de 25% en poids total de sucres tels que fructose, épilactose, galactose ou lactose relativement à la quantité de galactofructose.

Le galactofructose dans la méthode selon l'invention est incorporé dans une composition alimentaire. Par composition alimentaire on entend une composition destinée à l'alimentation de mammifères. Les mammifères sont de préférence des êtres humains, bien que l'invention puisse également être utile pour l'amélioration du bien-être d'animaux mammifères divers, tels que des animaux domestiques. La composition alimentaire est avantageusement une composition absorbée régulièrement, de préférence au moins une fois par mois, plus préférentiellement une fois par semaine, de manière particulièrement préférée quotidiennement. Des compositions alimentaires pour êtres humains telles que le pain, le beurre, le lait, les produits laitiers fermentés, les céréales, les biscuits, les boissons et jus de fruits sont avantageuses.

Dans un mode d'exécution particulièrement préféré de l'invention, les compositions alimentaires sont destinées à l'alimentation des enfants.

Dans un mode d'exécution avantageux de l'invention, la dose journalière absorbée, non moyennée, dépasse 0,1g, de préférence 0,5g. On recommande d'autre part qu'elle reste inférieure à 5g, dans certains cas inférieure à 2g.

Dans une variante préférée, les doses journalières de galactofructose sont comprises entre 0,1 et 5g, avantageusement entre 0,5 et 2g, pendant au moins 30 jours consécutifs. Dans cette variante, du galactofructose est absorbé régulièrement chaque jour et les doses ne sont plus des moyennes, mais des valeurs estimées quotidiennement.

L'effet régulateur du temps de transit intestinal selon l'invention se mesure concrètement par scintigraphie, le patient absorbant un traceur radioactif.

On a observé que la prise régulière selon l'invention de galactofructose en faibles doses permet non seulement d'accélérer le transit intestinal lorsqu'il est trop lent mais aussi de manière surprenante de le ralentir lorsqu'il est trop rapide, en particulier lorsqu'il est légèrement trop rapide. Dans l'invention, l'effet régulateur du temps de transit intestinal est tel que le temps de transit intestinal, mesuré par scintigraphie, est régulé à des valeurs généralement inférieures à 40 heures, souvent inférieures à 35 heures dans certains cas avantageux inférieures à 30 heures. Ces valeurs sont également généralement supérieures à 10 heures, souvent à 15 heures, dans certains cas avantageux supérieures à 20 heures.

Dans un mode de réalisation particulièrement surprenant de l'invention, le galactofructose utilisé pour son effet régulateur du temps de transit intestinal de mammifères, l'est pour ralentir le transit intestinal des mammifères qui l'absorbent (ces mammifères ayant typiquement un transit intestinal trop rapide avant de commencer l'absorption). Ainsi donc, un mammifère dont le transit intestinal est trop rapide avant d'avoir absorbé du galactofructose, voit son temps de transit intestinal, mesuré par scintigraphie, augmenté d'une valeur initiale donnée (avant absorption) à une valeur plus élevée, laquelle valeur plus élevée est généralement supérieure à 10 heures, souvent supérieure à 15 heures et dans certains cas avantageux supérieure à 20 heures, après qu'il ait absorbé du galactofructose incorporé dans une composition alimentaire selon la posologie conforme à la présente invention; la valeur après absorption conforme à la présente invention est par ailleurs généralement inférieure à 40 heures, souvent inférieure à 35 heures et dans certains cas avantageux inférieure à 30 heures. Selon ce mode de réalisation de l'invention, la valeur initiale (avant absorption) du temps de transit intestinal peut être par exemple à une valeur comprise dans une fourchette de 0 à 10 heures, dans une fourchette de 5 à 15 heures ou encore dans une fourchette de 10 à 20 heures. Selon ce mode de réalisation de l'invention, l'augmentation du temps de transit intestinal (valeur après utilisation conforme à l'invention moins valeur avant absorption), est avantageusement d'au moins 2 heures, de préférence d'au moins 5 heures ; elle peut être d'au moins 10 heures, voire d'au moins 15 heures.

Dans une variante particulièrement intéressante de ce mode de réalisation, le mammifère dont le transit intestinal est trop rapide avant d'avoir absorbé du galactofructose ne souffre néanmoins pas de diarrhée (son transit intestinal n'est que légèrement trop rapide), et il est même avantageusement sain, ne présentant aucun signe de maladie. La diarrhée peut être caractérisée par un temps de transit intestinal, mesuré par scintigraphie, d'au plus 8 heures, par exemple. Selon cette variante du second mode de réalisation de l'invention, la valeur initiale (avant absorption) du temps de transit intestinal du mammifère dont le transit intestinal est trop rapide avant d'avoir absorbé du galactofructose est de préférence égale à au moins 10 heures; de plus, ce mammifère voit son temps de transit intestinal augmenté, pour atteindre une valeur généralement supérieure à 15 heures, de préférence supérieure à 20 heures, après qu'il ait absorbé du galactofructose incorporé dans une composition alimentaire selon la posologie conforme à la présente invention. Selon cette variante du second mode de réalisation de l'invention, la valeur initiale (avant absorption) du temps de transit intestinal du mammifère dont le transit intestinal est trop rapide avant d'avoir absorbé du galactofructose vaut de manière particulièrement préférée une valeur comprise dans une fourchette de 10 à 20 heures; de plus, ce mammifère voit son temps de transit intestinal accru, pour atteindre une valeur généralement supérieure à 20 heures, de préférence une valeur entre 20 et 30 heures, après qu'il ait absorbé du galactofructose incorporé dans une composition alimentaire selon la posologie conforme à la présente invention.

Dans l'invention, le nombre des selles quotidien est généralement compris entre 0,5 et 1,5, pour une valeur normalisée de poids de selle unitaire de 220g/jour.

L'invention concerne également le galactofructose pour son effet régulateur du temps de transit intestinal de mammifères, combiné à son effet prébiotique sur la flore intestinale, lorsqu'il est incorporé dans une composition alimentaire et que celle-ci est absorbée par le mammifère en quantité correspondant à des doses journalières de galactofructose, moyennées sur 30 jours, comprises entre 0,1 et 5 g, avantageusement entre 0,1 et 2g.

Un prébiotique est un composé alimentaire non digestible, dégradable par les microorganismes de la flore intestinale. Cette dégradation provoque un effet sur la flore intestinale du mammifère, bénéfique pour sa santé car elle engendre un développement de certaines bactéries de la flore. Parmi celles-ci, les bifidobactéries sont connues pour avoir un rôle important par exemple sur le système immunitaire des hôtes. Leur développement sélectif grâce aux composés prébiotiques (effet prébiotique bifidogène) est donc bénéfique pour le bien être du mammifère hôte. On appelle donc effet prébiotique d'un composé non vivant, le changement, tant en composition qu'en activité, de la flore intestinale de l'hôte dû à l'ingestion de ce composé, lorsque ce changement a un effet bénéfique sur le bien-être et/ou la santé de l'hôte (voir : Gibson and Roberfroid, J. Nutr, 125, 1401, 1995*).*

Dans l'effet combiné selon l'invention, on observe, outre l'effet régulateur du transit intestinal, également une augmentation de la population de bifidobactéries dans la flore intestinale du mammifère hôte. Cette augmentation est d'au moins 50%. De préférence, la population est au moins doublée. De manière particulièrement préférée, la population en bifidobactéries *Bifidobacterium spp,* exprimée en log 10, est augmentée de 0,5 au moins, ce qui correspond à une multiplication de la population par un facteur d'environ 3 au moins. Les bifidobactéries *Bifidobacterium spp* protègent l'intestin contre la colonisation par des bactéries pathogènes. Cette protection résulte d'une part de la compétition à la surface des cellules, de la compétition pour les nutriments essentiels, de la production d'agents antimicrobiens et de la production de composés comprenant des acides gras à chaînes courtes, qui décroissent le pH des fèces et inhibent le développement de bactéries pathogènes. Les bifidobactéries *Bifidobacterium spp* sont également associées à un renforcement du système immunitaire, surtout chez les enfants, et à une action préventive contre le cancer, par réduction de l'activité d'enzymes qui convertissent des substances procancérigènes en substances cancérigènes (voir von Wright et al, Eur J. Gastroenterol.Hepatol. novembre 1999, 11(11), pp1195-1198).

Grâce à l'effet combiné selon l'invention, l'augmentation de la population de bifidobactéries ne requiert aucun probiotique dans la composition alimentaire. Il est même recommandé que la composition en soit sensiblement exempte. Par sensiblement exempte de probiotique on entend que la composition alimentaire seule, en l'absence de galactofructose, provoque une augmentation de la population de *Bifidobacterium spp* inférieure à 10%, généralement inférieure à 5%.

L'invention s'adresse à des mammifères en bonne santé, ne présentant aucun signe de maladie. Elle permet d'améliorer leur bien-être. Dans certaines circonstances, elle permet également de préserver leur bonne santé.

De même que pour le galactofructose selon l'invention, la composition alimentaire selon l'invention est avantageusement une composition absorbée régulièrement, et de préférence est destinée à l'alimentation des enfants, telle que le lait en poudre pour alimentation infantile.

Dans une variante d'exécution préférée de la composition alimentaire selon l'invention, celle-ci est également sensiblement exempte de probiotiques.

### Exemple

20 êtres humains volontaires, âgés de 18 à 50 ans, ayant un indice de masse corporelle compris entre 20 et 30 sont répartis de manière aléatoire en deux groupes de 10, l'un absorbant chaque jour, et ce pendant 30 jours, un verre de lait de 20cl produit au départ de lait en poudre additionné de 2,5g de poudre Solactis®, correspondant à 1,5g de galactofructose, et l'autre absorbant un placebo. La poudre de galactofructose a la composition suivante, en pourcentage relatif à la teneur en galactofructose:

| Tagatose | Fructose | Galactose | Epilactose | Lactose |
|---|---|---|---|---|
| <2% | <1% | <14% | <6% | <8% |

Les volontaires ne montrent aucun signe de maladie, en particulier de maladie intestinale. Ils n'ont jamais participé à un test portant sur les pré - ou probiotiques. Pendant le test ils n'absorbent aucun probiotique. Le temps de transit intestinal initial mesuré par scintigraphie est de 14h dans les deux groupes. A la fin du test (30 jours), le temps de transit est de 24h dans le groupe absorbant du galactofructose selon l'invention. Par contre, aucune variation significative (compte tenu des variations naturelles de mesure) en temps de transit n'est observée dans le groupe placebo.

## Revendications

1. Méthode non-thérapeutique pour ralentir le transit intestinal de mammifères sains, par administration d'une composition alimentaire comprenant du galactofructose, ladite composition alimentaire étant absorbée par le mammifère en quantité correspondant à des doses journalières non-moyennées de galactofructose comprises entre 0,1 et 5 g, avantageusement entre 0,1 et 2 g, pendant au moins 30 jours consécutifs, **caractérisé en ce que** le mammifère, tout en ne présentant aucun signe de maladie, présente néanmoins un transit intestinal légèrement trop rapide, le mammifère ayant un temps de transit intestinal avant absorption, mesuré par scintigraphie, égal à une valeur comprise dans une fourchette de 10 à 20 heures.

2. Méthode non-thérapeutique selon la revendication 1, **caractérisée en ce que** l'augmentation du temps de transit intestinal, mesuré par scintigraphie, est d'au moins 5 heures.

3. Méthode non-thérapeutique selon l'une quelconque des revendications précédentes, **caractérisée en ce que** le mammifère a un temps de transit intestinal après absorption, mesuré par scintigraphie, égal à une valeur comprise dans une fourchette entre 20 et 30 heures.

4. Méthode non-thérapeutique selon l'une quelconque des revendications précédentes, dans laquelle les doses journalières absorbées, non moyennées de galactofructose sont inférieures à 2 g.

5. Méthode non-thérapeutique selon l'une des revendications 1 à 4 **caractérisée en ce qu'**elle a en outre un effet prébiotique sur la flore intestinale.

6. Méthode non-thérapeutique selon l'une des revendications 1 à 5, **caractérisée en ce que** la composition alimentaire comprend moins de 25 % en poids total de sucres tels que fructose, épilactose, galactose ou lactose relativement à la quantité de galactofructose.

## Patentansprüche

1. Nicht-therapeutisches Verfahren zur Verlangsamung der Darmpassage von gesunden Säugetieren durch Verabreichung einer Galactofructose beinhaltenden Nahrungsmittelzusammensetzung, wobei die Nahrungsmittelzusammensetzung von dem Säugetier an mindestens 30 aufeinanderfolgenden Tagen in einer Menge aufgenommen wird, welche nichtgemittelten Tagesdosen von Galactofructose zwischen 0,1 und 5 g, vorzugsweise zwischen 0,1 und 2 g, entsprechen, **dadurch gekennzeichnet, dass** das Säugetier, ohne ein Krankheitsanzeichen aufzuweisen, eine etwas zu schnelle Darmpassage aufweist, wobei das Säugetier vor der Aufnahme eine durch Szintigrafie gemessene Darmpassagezeit entsprechend einem Wert in einer Spanne von 10 bis 20 Stunden aufweist.

2. Nicht-therapeutisches Verfahren gemäß Anspruch 1, **dadurch gekennzeichnet, dass** die Steigerung der durch Szintigrafie gemessenen Darmpassagezeit mindestens 5 Stunden beträgt.

3. Nicht-therapeutisches Verfahren gemäß irgendeinem der vorherigen Ansprüche, **dadurch gekennzeichnet, dass** das Säugetier nach der Aufnahme eine durch Szintigrafie gemessene Darmpassagezeit entsprechend einem Wert in einer Spanne von 20 bis 30 Stunden aufweist.

4. Nicht-therapeutisches Verfahren gemäß irgendeinem der vorherigen Ansprüche, wobei die aufgenommenen, nicht-gemittelten Tagesdosen von Galactofructose weniger als 2 g sind.

5. Nicht-therapeutisches Verfahren gemäß einem der Ansprüche 1 bis 4, **dadurch gekennzeichnet, dass** es unter anderem einen präbiotischen Effekt auf die Darmflora hat.

6. Nicht-therapeutsiches Verfahren gemäß einem der Ansprüche 1 bis 5, **dadurch gekennzeichnet, dass** die Nahrungsmittelzusammensetzung in der Zuckergesamtmenge weniger als 25% Zuckerstoffe wie Fructose, Epilactose, Galactose oder Lactose im Vergleich zur Menge der Galctofructose enthält.

## Claims

1. Non-therapeutic method for slowing down the intestinal transit of healthy mammals, by administering a food composition comprising galactofructose, said food composition being absorbed by the mammal in an amount corresponding to non-averaged daily doses of galactofructose comprised between 0.1 and 5g, advantageously between 0.1 and 2g, during at least 30 consecutive days, **characterized in that** the mammal, while not showing any signs of disease, shows a slighty too fast intestinal transit, the mammal having an intestinal transit time before galactofructose absorption, measured by scintigraphy, equal to a value ranging from 10 to 20 hours.

2. Non-therapeutic method according to claim 1, **characterized in that** intestinal transit time, measured by scintigraphy, is increased by at least 5 hours.

3. Non-therapeutic method according to any of the preceding claims, **characterized in that** the mammal has an intestinal transit time after galactofructose absorption, measured by scintigraphy, equal to a value ranging between 20 and 30 hours.

4. Non-therapeutic method according to any of the preceding claims, wherein absorbed daily doses, non-averaged, of galactofructose are less than 2 g

5. Non-therapeutic method according to any of claims 1 to 4, **characterized in that** it also has a prebiotic effect on the intestinal flora.

6. Non-therapeutic method according to any of claims 1 to 5, **characterized in that** the food composition comprises less than 25% by total weight of sugars selected from the group consisting of fructose, epilactose, galactose, and lactose, relative to the amount of said galactofructose.
